Europäisches Patentamt

European Patent Office

Office européen des brevets

(19)

(11) Veröffentlichungsnummer: **0 040 723**
**B1**

(12)     **EUROPÄISCHE  PATENTSCHRIFT**

(45) Veröffentlichungstag der Patentschrift:
**06.07.83**

(21) Anmeldenummer: **81103426.3**

(22) Anmeldetag: **06.05.81**

(51) Int. Cl.³: **C 07 C  29/84**, C 07 C  29/80,
C 07 C  31/02 // C07C31/04,
C07C31/08, C07C69/14,
C07C67/54, C07C7/05,
C07C9/15

(54) **Verfahren zur destillativen Zerlegung von aliphatische Alkohole enthaltenden Flüssigkeitsgemischen.**

(30) Priorität: **23.05.80  DE 3019766**

(43) Veröffentlichungstag der Anmeldung:
**02.12.81 Patentblatt 81/48**

(45) Bekanntmachung des Hinweises auf die Patenterteilung:
**06.07.83 Patentblatt 83/27**

(84) Benannte Vertragsstaaten:
**BE DE FR GB IT NL**

(56) Entgegenhaltungen:
**DE-A-955 237**
**US-A-2 604 440**
**US-A-3 268 572**

(73) Patentinhaber: **BASF Aktiengesellschaft,
Carl-Bosch-Strasse 38, D-6700 Ludwigshafen (DE)**

(72) Erfinder: **Kaibel, Gerd, Robert-Bosch-Strasse 4,
D-6840 Lampertheim (DE)**
Erfinder: **Hartmann, Horst, Lindenstrasse 45,
D-6737 Boehl-Iggelheim (DE)**
Erfinder: **Hochstein, Waldhelm, Dr., Am Wurmberg 4,
D-6713 Freinsheim (DE)**
Erfinder: **Mueller, Franz-Josef, Dr.,
Mueller-Thurgau-Weg 1, D-6706 Wachenheim (DE)**

## Verfahren zur destillativen Zerlegung von aliphatische Alkohole enthaltenden Flüssigkeitsgemischen

Die vorliegende Erfindung betrifft ein neues Verfahren zur destillativen Zerlegung von aliphatische Alkohole enthaltenden Flüssigkeitsgemischen in eine höher siedende alkoholhaltige Fraktion und eine tiefer siedende Fraktion der übrigen Flüssigkeiten.

Im einfachsten Fall besteht dieses Trennproblem in der Trennung eines Zweistoffgemisches, nämlich eines Alkohols und des tiefer siedenden Stoffes. Naturgemäß bereitet es keine Schwierigkeiten, wenn beide Komponenten deutlich verschiedene Siedepunkte haben oder wenn sie kein Azeotrop bilden. Sind diese Voraussetzungen jedoch nicht gegeben, so daß eine normale Fraktionierung nicht zum Erfolg führt, muß man sich technisch aufwendiger Methoden bedienen, z. B. der Fraktionierung unter abwechselnd erhöhtem und vermindertem Druck, von extraktiven Verfahren oder der Destillation mittels eines Hilfsstoffs. Diese Verfahren haben zudem den Nachteil, nur auf ganz bestimmte Fälle anwendbar zu sein. Ändert sich die Zusammensetzung des Stoffgemisches oder ist die Trennung neuer Stoffgemische erforderlich, so muß man meist auch nach einer neuen Methode suchen.

Was für das Zweikomponentensystem gilt, gilt allgemein auch für beliebige alkoholhaltige Stoffgemische, mit dem Unterschied, daß man eine alkoholhaltige und eine alkoholfreie tiefer siedende Fraktion erhält, von denen mindestens eine aus mehr als einer Komponente besteht, so daß sich gegebenenfalls weitere Trennverfahren anschließen müssen.

Der vorliegenden Erfindung lag die Aufgabe zugrunde, aliphatische Alkohole enthaltende Flüssigkeitsgemische in eine alkoholhaltige Fraktion und eine tiefer siedende Fraktion der übrigen Komponenten zu zerlegen, und zwar nach einem allgemein anwendbaren Verfahren in denjenigen Fällen, in welchen eine einfache Fraktionierung nicht zum Ziel führt.

Dementsprechend wurde gefunden, daß man aliphatische Alkohole enthaltende Flüssigkeitsgemische in einer Fraktionierkolonne destillativ in eine alkoholhaltige Fraktion und in eine tiefer siedende Fraktion der übrigen Komponenten zerlegen kann, wenn man die Fraktionierung in Gegenwart von Wasser und eines Alkanals sowie über etwa 3/4 der Kolonnenhöhe in Gegenwart einer nicht- oder schwerflüchtigen Säure vornimmt, oder wenn man anstelle der wäßrigen nicht- oder schwerflüchtigen Säure einen sauren Ionenaustauscher verwendet, den man im Mittelteil der Kolonne anordnet und daß man demgemäß das Wasser nicht in den oberen sondern in den unteren Kolonnenteil zurückführt.

Dieses Verfahren beruht auf dem Prinzip, den Alkohol im Mittelteil der Kolonne mit dem Alkanal intermediär als höhersiedendes Acetal oder Hemiacetal zu binden, so daß nur die leichter als der Alkohol siedenden Stoffe in den Oberteil der Kolonne gelangen können, wo sie dann über Kopf abgezogen werden können. Im unteren Kolonnenbereich wird das Acetal bzw. Hemiacetal wieder gespalten, so daß man dort ein Gemisch aus dem Alkohol, Wasser, dem Alkanal sowie gegebenenfalls weiteren höhersiedenden Komponenten erhält.

Ist das Alkanal leichter flüchtig als der Alkohol, so tritt es zum Teil als Kopfprodukt auf, wo es in einem weiteren Verfahrensschritt abgetrennt werden kann. Durch Wahl des Alkanals kann man es so einrichten, daß diese Trennung unproblematisch ist.

Wenn das Alkanal hingegen schwerer flüchtig ist als das Kopfprodukt, verbleibt es in der wäßrigen Sumpfphase, die in aller Regel auf konventionelle Weise in ihre Komponenten zerlegt werden kann.

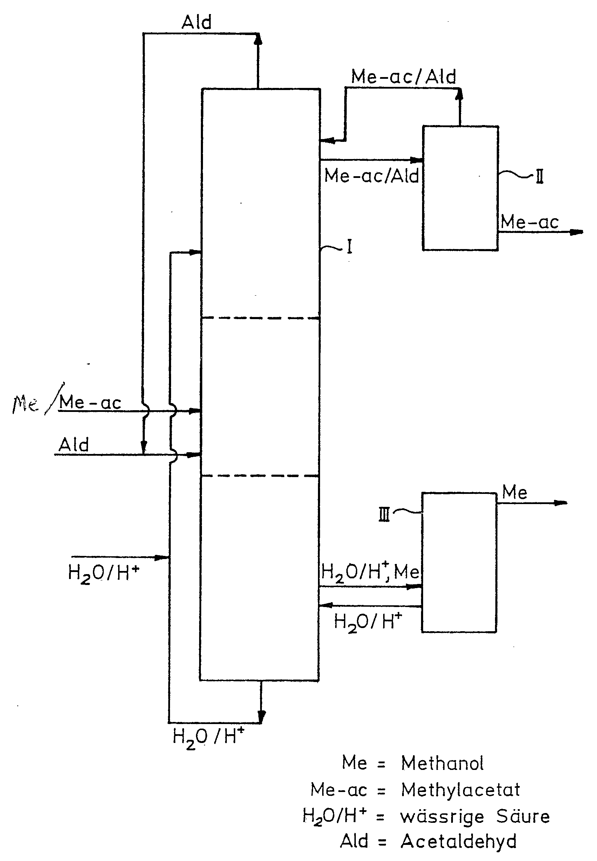

Das Verfahren sei anhand der Figur für den für die Erfindung typischen, technisch besonders wichtigen Fall der Trennung von Methylacetat und Methanol mittels eines leichter siedenden Alkanals erläutert.

Will man das Gleichgewicht der Veresterung von Essigsäure mit Methanol (Me) zugunsten des Methylacetats (Me-ac) verschieben, so muß man dieses laufend vom Reaktionsgemisch abdestillieren. Hierbei bilden sich jedoch ein Azeotrop aus 80 Gew.-% Methylacetat und 20 Gew.-% Methanol, welches in seine Komponenten zerlegt werden muß.

Hierzu gibt man dieses Azeotrop erfindungsgemäß an einer Stelle in die Kolonne I, in welcher die sauren Bedingungen herrschen, und zwar zweckmäßigerweise bei einer Temperatur, bei der das gesamte Methylacetat, nicht aber das gesamte Methanol verdampft. Diese Zulaufstelle liegt zweckmäßigerweise etwa in der Mitte der Kolonne.

Auf gleicher Höhe (zusammen mit dem azeotropen Gemisch oder getrennt davon) oder auch unterhalb, führt man das Alkanal zu, als welches man in diesem Falle vorteilhafterweise Acetaldehyd (Ald) verwendet. Etwa auf Höhe des dritten Viertels der Kolonne leitet man eine wäßrige Säure ($H_2O/H^+$) ein, welches auf dem Wege zum Kolonnensumpf die Bindung des Methanols an den Acetaldehyd bewirkt. Infolgedessen gelangt das praktisch methanolfreie Methylacetat zusammen mit einem Teil des Acetaldehyds in den oberen Kolonnenbereich. Das Methylacetat wird in einer Seitenkolonne II abgetrennt und der Acetaldehyd, der als Kopfprodukt anfällt, wird zweckmäßigerweise wieder in die Kolonne zurückgeführt.

Im Unterteil der Kolonne wird das Acetal bzw. das Hemiacetal wieder gespalten. Der Acetaldehyd entweicht nach oben, wo er zum Teil wieder frisches Methanol bindet, und im unteren Bereich erhält man eine wäßrig-saure Methanollösung. Aus dieser wird das Methanol in einer Seitenkolonne III wie üblich abdestilliert, und die wäßrige Säure wird zum Teil wieder in den Oberteil der Kolonne zurückgeführt.

Wie man erkennt, bilden wäßrige Säure und Acetaldehyd jeweils einen geschlossenen Kreislauf. Die Mengen dieser Stoffe brauchen daher nur um die unvermeidlich geringfügigen Verluste ergänzt zu werden.

Ähnlich gestaltet sich das Verfahren (wie nicht in die Figur eingezeichnet wurde), wenn man anstelle des Acetaldehyds ein höher als Methylacetat siedendes Alkanal wie etwa n-Butyraldehyd verwendet. In diesem Falle erhält man als Kopfprodukt fast ausschließlich das Methylacetat und im Sumpf eine wäßrig-saure Lösung, die Methanol und Butyraldehyd enthält. Hiervon braucht lediglich das Methanol in einer Seitenkolonne abdestilliert zu werden. Die verbleibende Lösung kann dann unmittelbar in den oberen Teil der Kolonne I zurückgeführt werden.

Eine vorteilhafte Ausführungsform des erfindungsgemäßen Verfahrens besteht entsprechend der zweiten Alternative des kennzeichnenden Teils von Anspruch 1 darin, anstelle der wäßrigen Säure einen sauren Ionenaustauscher zu verwenden, den man im Mittelteil der Kolonne fest anordnen kann. Aus verfahrenstechnischen Gründen empfiehlt es sich jedoch, das Stoffgemisch außerhalb der Kolonne I mit dem Ionenaustauscher in Berührung zu bringen, indem man einen Teil des Gemisches an einer oder mehreren Stellen abzieht, über den Ionenaustauscher leitet und auf gleicher Höhe wieder in die Kolonne zurückführt. Verwendet man einen Ionenaustauscher, so wird der Kreislauf der wäßrigen Säure durch einen Wasserkreislauf ersetzt.

Auf gleiche Weise, wie es für den Fall der Trennung von Methylacetat und Methanol erläutert wurde, wird das erfindungsgemäße Trennverfahren in den übrigen definitionsgemäßen Fällen vorgenommen. Voraussetzung ist lediglich, daß das betreffende Flüssigkeitsgemisch einen aliphatischen Alkohol einerseits sowie tiefer siedende Komponenten andererseits enthält und daß sich die tiefer siedenden Komponenten nicht durch einfache herkömmliche Fraktionierung von dem Alkohol abtrennen lassen. Hat man es mit mehreren tiefer siedenden und/oder mit mehreren höher siedenden Komponenten zu tun, so erhält man als Kopf- bzw. Sumpfprodukte jeweils Mehrkomponentengemische, deren Zerlegung nicht Gegenstand der Erfindung ist und daher nicht weiter erläutert zu werden braucht. Das gleiche gilt für den Fall, daß das Ausgangsgemisch zwei oder mehrere verschiedene Alkohole enthält. Hier würde sich u. U. die Aufgabe stellen, in anschließenden Verfahrensschritten diese Alkohole voneinander zu trennen.

Bis auf die Tatsache, daß das Trennverfahren über eine chemische Stufe, nämlich die intermediäre Acetalisierung, abläuft, handelt es sich um eine rein physikalische, also weitgehend stoffunabhängige Methode. Es erübrigt sich daher, alle Stoffgemische dieser Art, die in der Technik anfallen oder anfallen können im einzelnen aufzuzählen. In der Praxis handelt es sich bei den aliphatischen Alkoholen meist um $C_1-C_8$-Alkanole oder Alkohole, darunter vor allem Methanol, Ethanol, Propanol, Isopropanol und Butanole, Butenole und Methylbutenole. Diese Alkohole fallen bei verschiedenen chemischen Verfahren, z. B. Veresterungs- und Veretherungsverfahren, oder auch bei Löse- oder Extraktionsvorgängen im Gemisch mit Estern, Ethern, Ketonen, Alkenen und Alkanen an, die sich wegen eng benachbarter Siedepunkte nur unter erheblichem Aufwand fraktionieren und/oder durch Fraktionierung wegen Azeotropbildung überhaupt nicht abtrennen lassen. Das gleiche gilt selbstverständlich für den Fall, daß man nicht die sonstigen Stoffe, sondern die Alkohole als Begleitstoffe ansieht. Als Destillationshilfsstoff wählt man zweckmäßigerweise ein solches Alkanal, welches unter den Destillationsbedingungen gänzlich oder weitgehend flüchtig ist. Relativ zum Alkohol hochsiedende Alkanale sind wegen ihres geringen Dampfdrucks und damit wegen ihrer geringen Konzentration im Mittelteil der Kolonne weniger geeignet. Als Alkanale kommen daher vornehmlich $C_2-C_5$-Alkanale in Betracht, darunter vor allem Acetaldehyd, Propionaldehyd und n-Butyraldehyd. Formaldehyd ist wegen seiner Polymerisationsneigung hingegen nur bedingt geeignet.

Die Menge des Alkanals bemißt man zweckmäßigerweise so, daß für die Acetalisierung im Mittelteil der Kolonne pro Mol Alkohol mindestens 0,5 mol, vorzugsweise aber 0,8 bis 3 mol Alkanal zur Verfügung stehen.

Als Säuren kommen in erster Linie schwer- oder nichtflüchtige starke Mineralsäuren oder sulfonsäuren in Betracht, also z. B. Schwefelsäure, Phosphorsäure, Salpetersäure und p-Toluolsulfonsäure. Zweckmäßigerweise setzt man diese Säuren in Form ihrer 0,001 bis 2%igen wäßrigen Lösungen ein. Die Menge dieser Lösungen ist im allgemeinen so zu bemessen, daß im Mittelteil der Kolonne pro Liter Alkanol 0,5 bis 4 l der wäßrigen Säure zur Verfügung stehen.

Als Ionenaustauscher eignen sich vornehmlich Sulfonsäuregruppen tragende vernetzte Styrolpolymerisate wie sie z. B. unter den Bezeichnungen ®Lewatit SPL 108 und 118 und ®Lewasorb im Handel sind.

Die Menge des Wassers im Unterteil der Kolonne beträgt im allgemeinen 1 bis 5 l pro Liter des dort befindlichen Alkohols. Die vorstehenden Angaben über Art und Menge der eingesetzten Gemische und der Hilfsstoffe sind als Richtwerte zu verstehen. Aufgrund dieser Richtwerte und der erfindungsgemäßen Lehre lassen sich die Destillationsbedingungen jedoch für den konkreten Einzelfall unschwer ermitteln.

Dies gilt auch für das Temperaturprofil der Kolonne, für den Druck und für die destillationstechnischen Parameter des Verfahrens, wie Bauart der Kolonne, Anzahl der theoretischen Böden und das Rücklaufverhältnis. Je nach der Bildungsgeschwindigkeit des Acetals ist im Mittelteil der Kolonne eine bestimmte Verweilzeit einzuhalten. Ist diese kurz, kann der Mittelteil als Füllkörperkolonne mit 1 bis 30 theoretischen Böden ausgebildet sein, ist sie länger, so empfehlen sich hingegen Glocken- oder Ventilböden, da hier die Verweilzeit beliebig eingestellt werden kann. Für den Unterteil der Kolonne, in welchem praktisch keine Fraktionierung stattfindet, reicht prinzipiell ein theoretischer Boden aus, jedoch bevorzugt man hier im allgemeinen eine Bodenzahl von 5 bis 15. Das gleiche gilt für den Oberteil der Kolonne I sowie für die Nebenkolonnen II und III.

## Beispiel 1

### Trennung eines Methylacetat/Methanol-Azeotropes

In eine bei Normaldruck betriebene Füllkörperkolonne (Metallgeweberinge von 5 mm Durchmesser) von 200 cm Höhe, 4 cm lichter Weite und 45 theoretischen Böden wurden auf Höhe des 19. Bodens (von unten gerechnet) stündlich ein azeotropes Gemisch aus 107 g Methylacetat und 27 g Methanol sowie 63 g Acetaldehyd gegeben. Auf Höhe des 30. Bodens wurden der Kolonne stündlich 70 g 1 gew.-%ige wäßrige Schwefelsäure zugeführt.

Bei einem Rücklaufverhältnis von rd. 8 fielen am Kopf der Kolonne pro Stunde 63 g Acetaldehyd an. In einer als Abtriebskolonne geschalteten Seitenkolonne wurden stündlich 106 g Methylacetat gewonnen. Der Acetaldehyd wurde auf den 19. Boden der Kolonne zurückgeführt. Das Methylacetat war praktisch frei von Methanol. Auf Höhe des 10. Bodens wurde eine wäßrig-saure methanolische Lösung abgezogen, von der in einer Seitenkolonne stündlich ein Gemisch aus 27 g Methanol und 1 g Methylacetat abdestilliert wurde. Im Hinblick auf die Zielsetzung des Versuches, nämlich reines Methylacetat durch Veresterung von Essigsäure mit Methanol zu gewinnen, reicht die Reinheit des rückgewonnenen Methanols aus, da dieses in der Praxis wieder in die Veresterungsstufe zurückgegeben wird.

Die im Sumpf der Hauptkolonne anfallende wäßrige Säure wurde auf dem 30. Boden wieder in die Kolonne geleitet.

## Beispiel 2

### Trennung eines n-Pentan/Methanol-Gemisches

In der Apparatur von Beispiel 1 sowie auf die dort beschriebene Weise wurde stündlich ein Gemisch aus 84 g n-Pentan und 21 g Methanol mit Hilfe von 35 g Acetaldehyd und 80 g der wäßrigen Schwefelsäure quantitativ in seine Komponenten zerlegt.

## Beispiel 3

### Trennung eines Methylacetat/Methanol-Azeotrops

In der Apparatur von Beispiel 1 sowie auf die dort beschriebene Weise wurde stündlich ein azeotropes Gemisch aus 119 g Methylacetat und 30 g Methanol mit Hilfe von 50 g Propionaldehyd und 55 g der wäßrigen Schwefelsäure in eine Kopffraktion aus 118 g Methylacetat und 50 g Propionaldehyd und eine Sumpffraktion aus der wäßrigen Säure, 29,5 g Methanol und rd. 1 g Methylacetat zerlegt. Die Kopffraktion wurde durch einfache Fraktionierung in einer getrennten Kolonne in Propionaldehyd und in das noch etwas Methanol enthaltende Methylacetat zerlegt.

Aus der Sumpffraktion wurde sodann in einer Seitenkolonne das Methanol abgetrennt.

## Beispiel 4

### Trennung eines Methylacetat/Ethanol-Gemisches

In der Apparatur von Beispiel 1 sowie auf die dort beschriebene Weise wurde stündlich ein Gemisch aus 127 g Methylacetat und 12 g Ethanol mit Hilfe von 21 g Acetaldehyd und 80 g der wäßrigen Schwefelsäure bei einem Rücklaufverhältnis von 3 quantitativ in seine Komponenten zerlegt.

## Beispiel 5

### Trennung eines Methylacetat/Methanol-Gemisches

In der Apparatur von Beipsiel 1, jedoch ohne die beiden Seitenkolonnen, wurde stündlich ein Gemisch von 75 g Methylacetat und 37 g Methanol mit Hilfe von 37 g n-Butyraldehyd und 80 g wäßriger Schwefelsäure in eine Kopffraktion aus 72 g/h Methylacetat, 2,5 g/h Wasser und 0,5 g/h Methanol, sowie in eine Sumpffraktion aus der wäßrigen Säure, Methanol, Butyraldehyd, etwas Butyraldehyddimethylacetal und etwas Methylacetat zerlegt. Das Rücklaufverhältnis betrug etwa 4.

## Patentansprüche

1. Verfahren zur destillativen Zerlegung von aliphatische Alkohole enthaltenden Flüssigkeitsgemischen in eine höher siedende alkoholhaltige Fraktion und eine tiefer siedende Fraktion der übrigen Flüssigkeiten, dadurch gekennzeichnet, daß man

— die Fraktionierung in Gegenwart von Wasser und einem Alkanal sowie über etwa 3/4 der Kolonnenhöhe in Gegenwart einer nicht- oder schwerflüchtigen Säure vornimmt oder daß man

— anstelle der wäßrigen nicht- oder schwerflüchtigen Säure einen sauren Ionenaustauscher verwendet, den man im Mittelteil der Kolonne anordnet und daß man demgemäß das Wasser nicht in den oberen sondern in den unteren Kolonnenteil zurückführt.

2. Verfahren nach Anspruch 1, dadurch gekennzeichnet, daß man

a) das alkoholhaltige Ausgangsgemisch bei einer Temperatur in den Mittelteil der Kolonne gibt, bei der die tiefer siedende Fraktion gänzlich verdampft, nicht aber die höher siedende,

b) in den mittleren oder unteren Bereich der Kolonne ein Alkanal einführt, welches tiefer als der Alkohol siedet,

c) im oberen Viertel der Kolonne eine wäßrige Lösung der Säure einführt,

d) die tiefer siedende Fraktion über Kopf entnimmt oder, sofern diese Fraktion aus mehreren Komponenten besteht, ggf. wie üblich im oberen Kolonnenteil fraktioniert und das zusammen mit der tiefer siedenden Fraktion in den oberen Kolonnenteil gelangende Alkanal wie üblich abtrennt und in den Mittelteil der Kolonne zurückführt, und

e) die sich im Unterteil der Kolonne bildende alkoholhaltige wäßrige Fraktion von dort abzieht und in einer Seitenkolonne in den Alkohol bzw. in eine alkoholhaltige und eine wäßrige Fraktion zerlegt und letztere wieder in den Oberteil der Kolonne zurückführt.

3. Verfahren nach Anspruch 1, dadurch gekennzeichnet, daß man

a) das alkoholhaltige Ausgangsgemisch bei einer Temperatur in den Mittelteil der Kolonne gibt, bei der die tiefer siedende Fraktion gänzlich verdampft, nicht aber die höher siedende,

b) in den oberen Teil der Kolonne ein Alkanal einführt, welches höher als der Alkohol siedet,

c) im oberen Viertel der Kolonne eine wäßrige Lösung der Säure einführt,

d) die tiefer siedende Fraktion über Kopf entnimmt und wie üblich, gegebenenfalls mit Hilfe von Seitenkolonnen fraktioniert, und

e) die sich im Unterteil der Kolonne bildende alkohol- und alkanalhaltige wäßrig-saure Fraktion von dort abzieht und in einer Seitenkolonne eine Fraktionierung in eine alkoholhaltige Fraktion und ein Gemisch aus wäßriger Säure und Alkanal vornimmt und dieses Gemisch wieder in den oberen Teil der Kolonne zurückführt.

**Claims**

1. A process for the distillative separation of a liquid mixture, containing aliphatic alcohols, into a higher-boiling alcohol-containing fraction and a lower-boiling fraction of the other liquids, wherein the fractionation is carried out in the presence of water and of an alkanal and, over about 3/4 of the column height, in the presence of a non-volatile or only slightly volatile acid, or, in place of the aqueous non-volatile or only slightly volatile acid, an acidic ion exchanger is used, which is located in the middle zone of the column, and accordingly the water is returned to the lower zone of the column instead of to the upper zone.

2. A process as claimed in claim 1, wherein

a) the alcohol-containing starting mixture is introduced into the middle zone of the column at a temperature at which the lower-boiling fraction vaporizes completely but the higher-boiling fraction does not,

b) an alkanal which is lower-boiling than the alcohol is introduced into the middle or lower zone of the column,

c) an aqueous solution of the acid is introduced into the upper quarter of the column,

d) the lower-boiling fraction is taken off at the top or, if it consists of a plurality of components, is fractionated, if desired, in a conventional manner in the upper zone of the column, and the alkanal which passes, together with the lower-boiling fraction, into the upper zone of the column is separated off in a conventional manner and returned to the middle zone of the column, and

e) the alcohol-containing aqueous fraction which forms in the lower zone of the column is taken off from there and is separated, in a side stripper, into the alcohol or an alcohol-containing fraction, and an aqueous fraction, and the last-mentioned fraction is returned to the upper zone of the column.

3. A process as claimed in claim 1, wherein

a) the alcohol-containing starting mixture is introduced into the middle zone of the column at a temperature at which the lower-boiling fraction vaporizes completely but the higher-boiling fraction does not,

b) an alkanal which is higher-boiling than the alcohol is introduced into the upper zone of the column,

c) an aqueous solution of the acid is introduced into the upper quarter of the column,

d) the lower-boiling fraction is taken off at the top and is fractionated in a conventional manner, where appropriate with the aid of side strippers, and

e) the alcohol- and alkanal-containing aqueous acidic fraction which forms in the lower zone of the column is taken off from there and is fractionated, in a side stripper, to give an alcohol-containing fraction and a mixture of aqueous acid and alkanal, and this mixture is returned to the upper zone of the column.

**Revendications**

1. Procédé pour la décomposition de mélanges de liquides, comprenant des alcools aliphatiques, par distillation en une fraction alcoolique à point d'ébullition plus élevé et une fraction à plus bas point d'ébullition, formée par les autres liquides, caractérisé en ce que le fractionnement est réalisé

— en présence d'eau et d'un alcanal et, sur environ 3/4 de la hauteur de la colonne, en présence d'un acide non volatil ou peu volatil, ou
— en remplaçant l'acide aqueus non ou peu volatil par un échangeur d'ions acide, disposé dans la partie médiane de la colonne, l'eau étant recyclée, non dans la partie supérieure, mais dans la partie inférieure de la colonne.

2. Procédé suivant la revendication 1, caractérisé en ce que:

a) le mélange de départ, comprenant un alcool, est introduit dans la partie médiane de la colonne à une température, à laquelle la fraction à plus bas point d'ébullition est totalement vaporisée, tandis que la fraction à point d'ébullition plus élevé ne l'est pas;
b) un alcanal au point d'ébullition inférieur à celui de l'alcool est introduit dans la partie médiane ou la partie inférieure de la colonne;
c) une solution aqueuse d'acide est introduite dans le quart supérieur de la colonne;
d) la fraction à plus bas point d'ébullition est soutirée en tête de la colonne ou, dans le cas où cette fraction est constituée de plusieurs composants, fractionnée d'une manière usuelle dans la partie supérieure de la colonne, l'alcanal parvenu avec la fraction à plus bas point d'ébullition dans la partie supérieure de la colonne étant séparé de façon habituelle et recyclé dans la partie médiane de la colonne;
e) la fraction aqueuse alcoolique recueillie dans la partie inférieure de la colonne en est sourtirée et décomposée dans une colonne secondaire en alcool ou fraction alcoolique et phase aqueuse, cette dernière étant recyclée dans la partie supérieure de la colonne principale.

3. Procédé suivant la revendication 1, caractérisé en ce que:

a) le mélange de départ, comprenant un alcool, est introduit dans la partie médiane de la colonne à une température, à laquelle la fraction à plus bas point d'ébullition est totalement vaporisée, tandis que la fraction à point d'ébullition plus élevé ne l'est pas;
b) un alcanal au point d'ébullition supérieur à celui de l'alcool est introduit dans la partie supérieure de la colonne;
c) une solution aqueuse d'acide est introduite dans le quart supérieur de la colonne;
d) la fraction à plus bas point d'ébullition est soutirée en tête de la colonne et fractionnée éventuellement dans des colonnes auxiliaires;
e) la fraction aqueuse acide, renfermant de l'alcool et l'alcanal, recueillie dans la partie inférieure de la colonne, en est sourtée et soumise dans une colonne secondaire à un fractionnement en une fraction contenant l'alcool et un mélange d'acide aqueux et d'alcanal, ce mélange étant recyclé dans la partie supérieure de la colonne principale.

Ald

Me-ac/Ald

Me-ac/Ald

II

I

Me-ac

Me /Me-ac

Ald

Me

III

$H_2O/H^+$

$H_2O/H^+$,Me

$H_2O/H^+$

$H_2O/H^+$

Me = Methanol
Me-ac = Methylacetat
$H_2O/H^+$ = wässrige Säure
Ald = Acetaldehyd